# FASCICULE DE BREVET EUROPEEN

(11) **EP 2 954 072 B1**
(45) Date de publication et mention de la délivrance du brevet: **16.11.2016**
(21) Numéro de dépôt: 14702627.2
(22) Date de dépôt: 05.02.2014
(51) Int. Cl.: C12N 15/01, C12P 7/64, C12R 1/89, C12R 1/645, A23C 9/152, C11B 1/00, A23D 9/013, C11B 1/10, A23K 20/158

(54) **BIOMASSE DE LA MICROALGUE SCHIZOCHYTRIUM MANGROVEI ET SON PROCÉDÉ DE PRÉPARATION**
BIOMASSE AUS SCHIZOCHYTRIUM MANGROVEI-MIKROALGEN UND VERFAHREN ZUR HERSTELLUNG DAVON
BIOMASS OF THE MICROALGAE SCHIZOCHYTRIUM MANGROVEI AND METHOD FOR PREPARING SAME

(30) Priorité: 06.02.2013 FR 1351017
(43) Date de publication de la demande: 16.12.2015
(73) Titulaire: Roquette Frères, 62136 Lestrem (FR)
(72) Inventeur: COMINI, Serge, 59253 La Gorgue (FR); PORA, Bernard, Shanghai 200135 (CN)
(74) Mandataire: Cabinet Becker et Associés
(86) Numéro de dépôt international: PCT/EP2014/052214
(87) Numéro de publication internationale: WO 2014/122158

(56) Documents cités:
- EP-A1- 0 823 475
- WO-A1-2012/175027
- WO-A2-2007/068997
- WO-A2-2008/129358
- WO-A2-2013/010090
- KING-WAI FAN ET AL: "Lipid Characterization of Mangrove Thraustochytrid - Schizochytrium mangrovei", JOURNAL OF AGRICULTURAL AND FOOD CHEMISTRY, vol. 55, no. 8, avril 2007 (2007-04), pages 2906-2910, XP055069115, ISSN: 0021-8561, DOI: 10.1021/jf070058y
- CHODCHOEY KANOKWAN ET AL: "GROWTH, FATTY ACID PROFILE IN MAJOR LIPID CLASSES AND LIPID FLUIDITY OF AURANTIOCHYTRIUM MANGROVEI SK-02 AS A FUNCTION OF GROWTH TEMPERATURE", BRAZILIAN JOURNAL OF MICROBIOLOGY, SOCIEDADE BRASILEIRA DE MICROBIOLOGIA, SAO PAULO, BR, vol. 43, no. 1, janvier 2012 (2012-01), pages 187-200, XP009172362, ISSN: 1517-8382
- KAI-CHUANG CHAUNG ET AL: "Effect of culture conditions on growth, lipid content, and fatty acid composition of Aurantiochytrium mangrovei strain BL10", AMB EXPRESS, vol. 2, no. 1, janvier 2012 (2012-01), page 42, XP055078009, ISSN: 2191-0855, DOI: 10.1161/01.ATV.14.4.567

## Description

La présente invention se rapporte à une biomasse de microalgues riche en acide docosahexaénoïque (ou DHA), en acide palmitique et en phospholipides, notamment phosphatidylcholine, biomasse de microalgues du genre *Thraustochytrium,* plus particulièrement *Schizochytrium,* en l'occurrence la biomasse d'une souche de *Schizochytrium mangrovei* particulière.

Les lipides constituent une des trois grandes familles de macronutriments avec les protéines et les glucides.

Parmi les lipides, on distingue notamment les triglycérides et les phospholipides :
- Les triglycérides (également appelés triacylglycérols ou triacylglycérides ou TAG) sont des glycérides dans lesquels les trois groupements hydroxyle du glycérol sont estérifiés par des acides gras. Ils sont le constituant principal de l'huile végétale et des graisses animales.

Les triglycérides représentent environ 95 % des lipides alimentaires ingérés par l'Homme. Dans l'organisme, ils sont présents principalement dans les tissus adipeux et constituent la forme principale de stockage de l'énergie.
- Les phospholipides sont des lipides amphiphiles, c'est-à-dire constitués d'une « tête » polaire (hydrophile) et de deux « queues » aliphatiques (hydrophobes).

Les phospholipides sont des lipides de structure car ils sont des constituants des membranes cellulaires dont ils assurent entre autre la fluidité.

La plupart des phospholipides sont les phosphoglycérides, dont la tête s'organise autour d'un résidu glycérol-3-phosphate estérifié par une molécule polaire, et les deux queues sont les chaînes aliphatiques de deux acides gras.

Les autres phospholipides sont les sphingomyélines, qui dérivent structurellement de la sphingosine et non du glycérol, la sphingosine constituant l'une des deux queues aliphatiques.

Les premiers phospholipides isolés de tissus vivants ont été caractérisés à partir de lécithine de jaune d'oeuf - il s'agissait plus précisément de phosphatidylcholines. C'est d'ailleurs la raison pour laquelle les phosphatidylcholines sont également connues sous le nom de lécithines.

Les phosphatidylcholines sont naturellement produites par le foie. Elles sont un important constituant de la bile dans laquelle elles émulsionnent les graisses présentes dans le duodénum. Elles sont aussi nécessaires, en plus des sels biliaires, pour empêcher que les gouttelettes lipidiques ne se ré-agglutinent.

En tant que phospholipides, les phosphatidylcholines participent à la membrane des cellules et servent à préserver leur viscoélasticité. Elles sont une composante essentielle du système nerveux et constituent près de 30 % du poids sec du cerveau et 15 % des nerfs.

Triglycérides et phospholipides sont composés majoritairement d'acides gras qui sont à la fois apportés par l'alimentation et, pour certains d'entre eux, synthétisés par l'organisme.

La classification biochimique (basée sur le nombre de doubles liaisons contenues dans la molécule d'acide gras) distingue les acides gras saturés (AGS), les acides gras monoinsaturés (AGMI) et les acides gras polyinsaturés (AGPI).

Du point de vue physiologique, on distingue :
- les acides gras indispensables nécessaires au développement et au bon fonctionnement du corps humain, mais que notre corps ne sait pas fabriquer ;
- les acides gras dit « conditionnellement » indispensables, essentiels pour la croissance normale et les fonctions physiologiques des cellules mais qui peuvent être fabriqués à partir de leur précurseur s'il est apporté par l'alimentation. Ils sont donc rigoureusement requis si leur précurseur indispensable est absent.
- Les acides gras non indispensables.

L'ensemble des acides gras indispensables et « conditionnellement » indispensables constituent les acides gras essentiels.

Les autres acides gras sont dits non essentiels.

Parmi les acides gras non indispensables, on trouve notamment :
- l'acide eicosapentaénoïque (EPA) de la famille des acides gras oméga 3,
- l'acide oléique, l'acide gras monoinsaturé majoritaire dans notre alimentation, et
- les acides gras saturés, tels l'acide laurique, l'acide myristique ou l'acide palmitique.

Les acides gras polyinsaturés sont classés en fonction de la position de la première double liaison, à partir de la fonction méthyle finale.

Ainsi, dans la nomenclature, pour oméga « x » ou « nx », « x » correspond à la position de la première insaturation.

On distingue deux grandes familles d'acides gras essentiels : les acides gras oméga 6 (ou AGPI n-6), dont le précurseur et le représentant majeur est l'acide linoléique (LA) et les acides gras oméga 3 (ou AGPI n-3) dont le précurseur est l'acide alpha-linolénique (ALA).

La majorité des acides gras polyinsaturés d'intérêt biologique appartient à la famille des oméga 6 (acide arachidonique ou ARA) ou oméga 3 (acide eicosapentaénoïque ou EPA, acide docosahexaénoïque ou DHA).

En outre, dans la nomenclature, on définit également le nombre de carbone constituant la chaîne ; ainsi l'EPA est décrit comme C20:5 et le DHA comme C22:6.

Le « 5 » et « 6 » correspondent ainsi au nombre d'insaturations de la chaîne carbonée présentés respectivement par l'EPA et par le DHA.

Le DHA, de la famille des acides gras oméga 3, est un acide gras que l'organisme sait synthétiser à partir de l'acide alpha-linolénique, ou qui est apporté par la consommation de poissons gras (thon, saumon, hareng...).

Le DHA joue un rôle important dans la structure des membranes et dans le développement et le fonctionnement du cerveau et de la rétine.

Les huiles de poisson sont utilisés principalement comme source d'acides gras de type oméga 3, tels le DHA et l'EPA, mais on les trouve également dans les huiles de microalgues où on les extrait soit en mélange, soit séparément, comme c'est le cas par exemple des huiles issues de certaines souches sélectionnées, telles que celles du genre *Schizochytrium,* qui ne contiennent que des traces d'EPA mais de fortes teneurs en DHA.

Des préparations commerciales de biomasse de microalgues riches en DHA sont disponibles.

On peut citer ainsi, par exemple :
- proposés pour l'alimentation en aquaculture des rotifères, des produits de la gamme ALGAMAC, commercialisé par AQUAFAUNA BIO-MARINE Inc., ou
- des produits commercialisés par la société DSM sous le nom de marque DHA GOLD™.

Cependant, il demeure un besoin de disposer de biomasses de microalgues de qualité, à haute tenue en DHA, et présentant des profils tout à fait particulier en acides gras saturés ou polyinsaturés à longue chaîne et en phospholipides.

Il a été tout d'abord du mérite de la société Demanderesse de proposer une nouvelle biomasse de microalgue, riche en DHA, présentant :
- de faibles teneurs en acide gras polyinsaturés autres que le DHA (tel que l'EPA),
- une teneur limitée en certains acides gras saturés à longue chaîne (tels que l'acide myristique et l'acide laurique).
- une teneur élevé en phospholipides (jusqu'au double des quantités classiquement trouvées dans les préparations commerciales), plus particulièrement en phosphatidylcholine.

Soucieuse également de mettre au point un procédé de production bien plus efficace et bien moins couteux que ceux décrits dans l'état de la technique, la société Demanderesse a, au cours de ses recherches, identifié une nouvelle souche de *Schizochytrium mangrovei* productrice de DHA présentant la particularité de produire :
- très peu d'acides gras saturés hypercholesterolémiants (moins de 6 % d'acides laurique et myristique dont l'homme du métier sait qu'ils sont les plus hypercholestérolémiants connus),
- plus de 40 % d'acide palmitique,
(les % s'entendant ici en poids d'acides gras totaux).

L'acide palmitique, également appelé acide hexadécanoïque ou acide cétylique, est l'un des acides gras saturés en C16:0 les plus courants chez les animaux et les plantes.

L'acide palmitique est le premier acide gras produit au cours de la lipogenèse ; à partir de lui, des acides gras plus longs peuvent être produits.

De plus, il est l'acide gras utilisé préférentiellement pour synthétiser de l'ATP. Le bilan énergétique de sa combustion indique 129 ATP. Il constitue ainsi un excellent aliment énergétique.

Industriellement on utilise également l'acide palmitique pour la fabrication des margarines, des savons durs.

Dans le domaine des peintures, étant donné qu'il est saturé, l'acide palmitique ne peut pas polymériser et se rigidifier une fois en contact avec l'oxygène de l'air (à la différence de l'acide oléique, linoléique et linolénique). Il reste donc sous sa forme de solide mou et agit (avec l'acide stéarique) comme plastifiant des liants huileux polymérisés. Ainsi, avec l'acide stéarique, il assure l'élasticité nécessaire à la bonne conservation des matières picturale à l'huile, à travers le temps.

Par ailleurs, la biomasse de *Schizochytrium mangrovei* selon l'invention présente :
- une teneur en phospholipides comprise entre 1,5 et 2%, dont 1 à 1,3 % constitués de phosphatidylcholine,
- une teneur en acides aminés totaux comprise entre 10 et 20 % exprimé en N 6,25,
(les % s'entendant ici en poids sur 100 g de biomasse à 99 % de matière sèche).

Cette souche de *Schizochytrium mangrovei* a été déposée en France le 22 novembre 2012 auprès de la Collection Nationale de Cultures de Microorganismes de l'Institut Pasteur (CNCM), 25 rue du docteur Roux, 75724 Paris Cedex 15, France, sous le numéro CNCM I-4702.

Elle a été caractérisée par séquençage des gènes codant pour l'ARNr 18 S : ce qui a permis de l'identifier comme étant une souche du type *Schizochytrium mangrovei.*

Par conséquent, la présente invention est relative à la souche de *Schizochytrium mangrovei déposée* le 22 novembre 2012 auprès de la CNCM sous le numéro I-4702.

Cette souche pourra être désignée « CNCM I-4702» ultérieurement dans la présente demande.

La présente invention concerne également un variant de cette souche ou une souche dérivée de celle-ci, ledit variant ou ladite souche dérivée conservant la propriété de produire des teneurs en DHA et acide palmitique élevées.

En particulier, elle concerne une souche de *Schizochytrium mangrovei* obtenue à partir de la souche CNCM I-4702 par mutagenèse ou par transformation génique. La mutagenèse peut être dirigée et/ou aléatoire. Cette souche conserve la propriété de produire des teneurs en DHA et acide palmitique élevées. Notamment, elle est capable de produire plus de 35 % de DHA et plus de 40 % d'acide palmitique, ces deux % étant exprimés en poids d'acides gras totaux, notamment lorsqu'elle est cultivée dans les conditions décrites dans l'exemple 1. En outre, elle produit entre 1 et 1,3 % de phosphatidylcholine, exprimé en poids de biomasse à 99 % de matière sèche.

La présente invention concerne aussi une méthode de préparation d'une telle souche comprenant la mutagenèse ou la transformation génique de la souche CNCM I-4702 et facultativement une étape de criblage permettant de sélectionner les souches produisant :
- plus de 35 % de DHA,
- plus de 40 % d'acide palmitique,
   (ces deux % exprimés en poids d'acides gras totaux).
- entre 1 et 1,3 % de phosphatidylcholine,
   (ce % exprimé en poids de biomasse à 99 % de matière sèche)

L'invention concerne une méthode de culture de la souche CNCM I-4702 ou d'un variant de celle-ci conservant la capacité de production de DHA et d'acide palmitique, comprenant une étape de culture de cette souche dans un milieu approprié, dans des conditions de fermentation adaptées.

L'invention concerne par ailleurs une méthode de préparation de la biomasse de *Schizochytrium mangrovei,* caractérisée en ce qu'elle est préparée par la succession d'étapes suivantes :
∘ culture de la souche en conditions hétérotrophiques de manière à produire une biomasse présentant entre 35 et 40 % de DHA, exprimé en poids d'acides gras totaux, entre 40 et 50 % en poids d'acide palmitique, exprimé en poids d'acides gras totaux, et entre 1 et 1,3 % de phosphatidylcholine, % exprimé en poids de biomasse,
∘ récolte de la biomasse ainsi préparée
∘ séchage de ladite biomasse.

La culture est réalisée en conditions hétérotrophiques. Généralement, l'étape de culture comprend une étape de préculture, pour revivifier la souche, puis une étape de culture ou de fermentation proprement dite. Cette dernière étape correspond à l'étape de production des composés lipidiques d'intérêt.

La société Demanderesse recommande, pour la souche CNCM I-4702 de mettre en oeuvre une fermentation aérobie en trois étapes, comme il sera exemplifié ci-après.

Après une étape préalable de préculture, les trois étapes de fermentation sont caractérisées par une culture de la souche CNCM I-4702 dans un milieu dans lequel l'apport en sources de carbone est régulé en fonction de la consommation en glucose par le microorganisme.

Il est ainsi noté que la consommation en glucose est consommé de façon graduelle dans les premières heures de fermentation, puis elle se maintient constante jusqu'à la fin de la fermentation, comme il sera exemplifié ci-après.

La présente invention concerne ensuite la récupération, en fin de fermentation, de la biomasse riche en composés lipidiques d'intérêt, en l'occurrence ici le DHA et l'acide palmitique.

Après l'étape de fermentation, la biomasse est:
- pasteurisée, de manière à inactiver les enzymes de dégradation des lipides (lipases) présentes dans la biomasse en tant que telle, mais aussi dans le milieu de culture,
- récupérée du milieu de fermentation par toute méthode connue en soi de l'homme du métier, par exemple la biomasse peut être extraite du fermenteur et simplement concentrée par microfiltration ou centrifugation, ou lavée par succession de concentrations-dilutions avec une solution aqueuse.

Après fermentation, la biomasse peut contenir :
- entre 35 et 40 % de DHA, exprimé en poids d'acides gras totaux,
- entre 40 et 50 % en poids d'acide palmitique, exprimé en poids d'acides gras totaux,
- entre 1,5 et 2% de phospholipides, dont 1 à 1,3 % constitués de phosphatidylcholine, % exprimé en poids de biomasse,

La présente invention concerne enfin l'utilisation de la biomasse riche en DHA, acide palmitique et phosphatidylcholine produite par l'un quelconque des procédés de la présente invention, dans la préparation de compositions destinées aux domaines alimentaires, notamment nutrition animale, mais également humaine.

Ainsi, elle concerne une méthode de préparation de compositions destinées aux domaines alimentaires comprenant la production d'une biomasse riche en DHA, en acide palmitique et en phosphatidylcholine par l'un quelconque des procédés de la présente invention puis la préparation de compositions destinées aux domaines alimentaires.

La présente invention concerne en particulier un produit ou une composition comprenant la souche CNCM I-4702 ou un variant de celle-ci conservant la capacité de production du DHA, et une biomasse obtenue après culture ou fermentation de celle-ci.

De préférence, ce produit ou cette composition est une composition alimentaire ou un complément alimentaire ou nutritionnel.

Il peut être sous forme liquide ou solide.

Ce produit ou cette composition peut être sous forme de poudre, granule, gélule, capsule, ou cachet, de préférence sous forme de poudre.

L'invention sera mieux comprise à l'aide des exemples qui suivent, lesquels se veulent illustratifs et non limitatifs.

### Exemple 1 : Production d'une biomasse riche en DHA et en acide palmitique par la souche de Schizochytrium mangrovei CNCM I-4702

Les compositions des milieux de culture et les conditions de fermentation sont présentées dans les tableaux suivants.

**Tableau 1 : Composition des milieux de culture**

| Fermenteur successifs de : | 100 litres | 1 m³ | 10 m³ |
|---|---|---|---|
| Volume efficace du fermenteur | 70 litres | 700 litres | 7000 litres |
| | | | |
| Glucose (kg) | 6 | 41,3 | 1120 |
| Gluconate mono sodique (kg) | 4,494 | 26,67 | / |
| « Corn steep » liquide (kg) | / | / | 119 |
| Extraits de levures (kg) | 0,448 | 2,03 | 42 |
| NaCl (kg) | 1,4 | 2,66 | 16,8 |
| KH2PO4 (kg) | 0,448 | 2,80 | 33,6 |
| MgSO4 (kg) | 1,6 | 7,35 | 42 |
| CaCl2 (kg) | 0,02 | 0,14 | 4,2 |
| NaHCO3 (kg) | 0.,2 | 0,07 | 4,2 |
| Agent antimousse (kg) (DOW FAX DF 104) | 0,112 | 1,12 | 11,2 |
| Na2SO4 (kg) | 0,02 | 0,07 | 42 |
| Urée (kg) | / | / | 18,9 |
| KCl (kg) | / | / | 2,8 |
| Ammoniaque (28%, litre) | / | / | 33,6 |
| liquide « rouge » (litre) | 0,098 | 0,98 | 14 |
| Liquide « vert » (litre) | 0,140 | 1,4 | 19.60 |

La concentration initiale en glucose dans le milieu de culture stérilisé du fermenteur de 10 m³ est fixée à 15 à 16 g/l.

**Tableau 2. Paramètres de contrôle de la préculture et des 3 fermentations successives.**

| | préculture | 100 litres | 1 m³ | 10 m³ |
|---|---|---|---|---|
| Volume de charge | 200 ml x3 | 70 litres | 0,7 m³ | 7 m³ |
| Température (°C) | 28°C | 28°C | 28°C | 0 - 68 h: 28°C 68 - 80 h : 26°C |
| pH | Pas de | Pas de | Pas de régulation | Régulation à 6.2 ~ |
| Débit d'air (m³/h) | - | 4,5 - 5,0 | 41 - 42 | 0 - 24 h : 95-105; 24 - 60 h : 50-60; 60 - 80 h: 25-30 |
| Pression (Mpa) | - | 0,025 - 0,03 | 0 ,025 - 0,03 | 0,03 |
| Agitation ( rpm) | 180rpm | 120-140 | 140 | 70 - 75 |
| Durée de la fermentation (h) | 48 | 24 - 48 | 24 - 48 | ∼ 80 |

Le principe d'alimentation en glucose durant la fermentation en 10 m³ est le suivant :
✔ la concentration de la solution d'alimentation en glucose est de 50 - 60%,
✔ Quand la concentration résiduelle en glucose (RCS) diminue à 1 g/l avant 48 h, on alimente avec une solution à 0,65 g/l de glucose en une fois jusqu'à épuisement du glucose et l'achèvement de la fermentation.

Les résultats des différentes fermentations sont présentés dans les tableaux suivants.

**Tableau 3. Première fermentation**

| Temps (h) | 0 | 8 | 16 | 23 |
|---|---|---|---|---|
| pH | 5,6 | 5,6 | 7,0 | 7,6 |
| Concentration résiduelle en glucose (g/100ml) | 5,4 | / | / | 4,7 |
| Azote aminé (mg/100 ml) | 269 | / | / | 123 |
| Phosphore (ppm) | 692 | / | / | 159.1 |
| Poids sec de cellules (g/L) | ∼0 | / | / | 29 |

**Tableau 4. Seconde fermentation**

| Temps (h) | 0 | 6h | 10h | 14h | 18h | 22h | 24h |
|---|---|---|---|---|---|---|---|
| pH | 5,4 | 7,1 | 7,6 | 8,0 | 8,1 | 8,05 | 7,9 |
| Concentration résiduelle en glucose (g/100ml) | 5,8 | / | / | / | / | / | 2,2 |
| Azote aminé (mg/100ml) | 166 | / | / | / | / | / | 75 |
| Phosphore (ppm) | 725,8 | / | / | / | 141,2 | / | 86,7 |
| Poids sec de cellules (g/L) | 1,6 | / | / | / | / | / | 46,7 |

**Tableau 5. Troisième fermentation**

| Temps (h) | 0 | 12 | 24 | 36 | 48 | 60 | 64 | 72 | 81 |
|---|---|---|---|---|---|---|---|---|---|
| pH | 6,7 | 6,3 | 5,8 | 6 | 6,2 | 6,45 | 6,5 | 6,5 | 6,6 |
| Concentration résiduelle en glucose (d/100ml ) | 14,1 | 12,1 | 9,1 | 11,2 | 9,6 | 5,8 | 5,8 | 4,35 | 1,35 |
| Azote aminé (mg/100ml) | 165 | 84 | 53 | 36 | 35 | 39 | / | 37 | 31 |
| Débit d'air (m³/h) | 95 - 100 m³/h | | | 60 m³/h | | | 30 m³/h | | |
| Température (°C) | 28 | | | | | | | 26 | |
| Phosphore (ppm) | 724,1 | 692,4 | 661,3 | 544,3 | 599 | 659 | / | 651,2 | 618 |
| Poids sec de cellules (g/L) | 11,8 | 25,7 | 37,4 | 38,5 | 44,2 | 69,9 | / | 75,4 | 80,5 |
| Contenu en DHA (%) | / | / | / | 30,7 | 27,6 | 34 | / | 32,75 | 31,4 |

Presque 70 % de l'azote aminé est consommé durant les premières 24 h de fermentation ; le phosphore l'est également durant l'étape de croissance cellulaire et n'est plus consommé ensuite (en lien avec la régulation du débit d'aération et la température de fermentation).

Le taux d'accumulation des lipides et le taux de production en DHA augmente progressivement et atteint un maximum à 72 heures.

La récupération des cellules est donc optimale dès ce temps de fermentation.

Il est choisi d'arrêter la fermentation à 81 h.

### Exemple 2 : Récupération et conditionnement de la biomasse de la souche CNCM I-4702 pour des applications en alimentations animale et humaine

La biomasse récupérée au terme de la fermentation décrite dans l'exemple 1 présente la composition suivante :

**Tableau 6.**

| Volume récupéré (m³) | Poids sec en cellules (g/L) | Contenu en DHA (%) |
|---|---|---|
| 7.5 | 80.5 | 31.4 |

La biomasse récupérée est centrifugée une première fois à 6000 g, les cellules récupérées sont ensuite diluées dans de l'eau stérile (rapport 1,5/1) puis centrifugées une seconde fois.

On la soumet ensuite à un traitement thermique à 70°C pendant 15 minutes.

On récupère 2,55t de biomasse humide (16,7 % de matière sèche).

On lui ajoute, pour les formules destinées à l'alimentation animale :
- 2% de maltodextrine d'un DE (Dextrose Equivalent) de 18,
- 0,5% de mono et diglycérides,
- 1% d'acide citrique,
- 0,2% d'antracyne 2727 (comme antioxydant) et
- 0,2% de phosphate tricalcique.

Pour les formules destinées à l'alimentation humaine, des antioxydants alimentaires de type tocophérols ou extraits de romarin sont utilisés.

On atomise cette biomasse en atomiseur simple effet (conduite classique connue de l'homme du métier) dans les conditions présentées dans le tableau 7 suivant :

**Tableau 7.**

| Paramètres | Valeurs |
|---|---|
| Température de la solution | 65 - 70°C |
| Température d'entrée de l'air | 155 - 160°C |
| Température de sortie de l'air | 75 °C- 80°C |
| Pression | ∼ 7.5 Mpa |
| Entrée de biomasse humide (kg) | 690,7 |
| Matière sèche de la biomasse (%) | 16,7 |
| Poids théorique de cellules (kg) | 115,35 |
| Poids de cellules sèches obtenues (kg) | **144,03** |
| Rendement de séchage (%) | 125 |

La composition de la biomasse séchée est la suivante (tableau 8) :

**Tableau 8.**

| Paramètres | Valeurs |
|---|---|
| Contenu en DHA (% sur acides gras totaux) | 35,2 |
| Protéines N6,25 en g/100 g brut | 16,7 |
| Phospholipides (%) | 1,6 |
| Tenue en eau résiduelle (%) | 1,2 |
| Cendres (%) | 6,4 |
| POV (meq/kg) | 0,4 |
| P- Anisidine (%) | 23,8 |

### Exemple 3. Etude comparative du profil lipidique d'une biomasse conforme à l'invention en regard de celles du commerce

Les acides gras ont été déterminés par chromatographie en phase gazeuse sous la forme d'esters méthyliques après transestérification au méthanol chlorhydrique et extraction au chloroforme. Les résultats sont exprimés en distribution, en % ; l'analyse est réalisée par la méthode de normalisation interne.

Un chromatographe (Type VARIAN 3800) équipé d'un injecteur « split-splitless » muni d'un « tapfocus liner » et d'un détecteur à ionisation de flamme a été utilisé.

Une solution d'étalon interne à environ précisément 0.5 mg de méthylheptadécanoate par ml de méthanol a été préparée. Le méthylheptadécanoate a servi de repère chromatographique.

Dans un tube de 6 ml, on a pesé environ précisément 30 mg d'échantillon séché au préalable. On a ajouté à la pipette à 2 traits 1 ml de la solution d'étalon interne puis 2 ml de méthanol chlorhydrique 3N. On a ensuite bouché et placé au bain à sec thermostaté à 110°C pendant 4 h.

Après refroidissement, on a ajouté environ 0.5 ml d'eau et 0.5 ml d'eau saturée en chlorure de sodium, extraire par 3 fois 1 ml de chloroforme. On a récupéré les phases chloroformiques dans un tube de 6 ml en les séchant sur une colonne contenant du sulfate de sodium. On a concentré sous courant d'azote jusqu'à 1 mL environ et injecté.

La distribution de chaque acide gras (i), en %, a été obtenu par le ratio de la surface du pic de cet acide gras par rapport à la somme des surfaces de tous les pics repérés sur le chromatogramme, de l'acide laurique (C12:0) au DHA (C22:6 Δ4c, 7c, 10c, 13c, 16c, 19c) inclus, en excluant le pic du méthylheptadécanoate.

Les phospholipides sont analysés après le cassage et l'extraction à froid de la biomasse réalisés dans les conditions suivantes.

### Cassage de la biomasse

Dans un tube pyrex à visser, on pèse précisément 200 mg de biomasse fraîche. On ajoute environ 1-1,5 cm de billes verre (type RETSCH de référence 22.222.0003) et 0,1 ml de méthanol. On ferme hermétiquement le tube et agite au moyen d'un agitateur de type vortex pendant au moins 5 min.

### Extraction à froid

Dans une petite nacelle en aluminium, on pèse précisément 2 mg de Triphényl Phosphate (pureté ≥ 98 %) à la balance au microgramme.

On place la nacelle dans un tube RMN en pyrex de 5 mm de diamètre ainsi que 0,9 ml de Méthanol et 2 ml de chloroforme. On ferme hermétiquement le tube et agite au moyen de l'agitateur vortex pendant 1 min.

On place le tube au réfrigérateur. Après décantation (au minimum 1 heure), on récupère délicatement la phase supérieure limpide et la transfère dans un godet en verre pour évaporation à sec, à température ambiante, sous courant d'azote.

On solubilise l'extrait sec dans 0,5 ml de CDCl₃ et 0,1 ml de CD₃OD et transfère dans un tube RMN.

Pour exprimer la teneur en phospholipides à partir de la teneur en phosphore obtenue par RMN on prend en compte le phosphore apporté par les quatre principaux phospholipides et utilise l'acide oléique pour calculer la masse molaire de chacun d'entre eux.

La teneur en phospholipides est égale à la somme des quantités de ces quatre phospholipides ainsi calculées. Les biomasses analysées selon ces méthodes (dans les tableaux 9 et 10 suivants), outre celle de l'invention, sont des biomasses commercialisées par les sociétés AQUAFAUNA BIO-MARINE Inc., DSM/MARTEK et NEW HORIZON

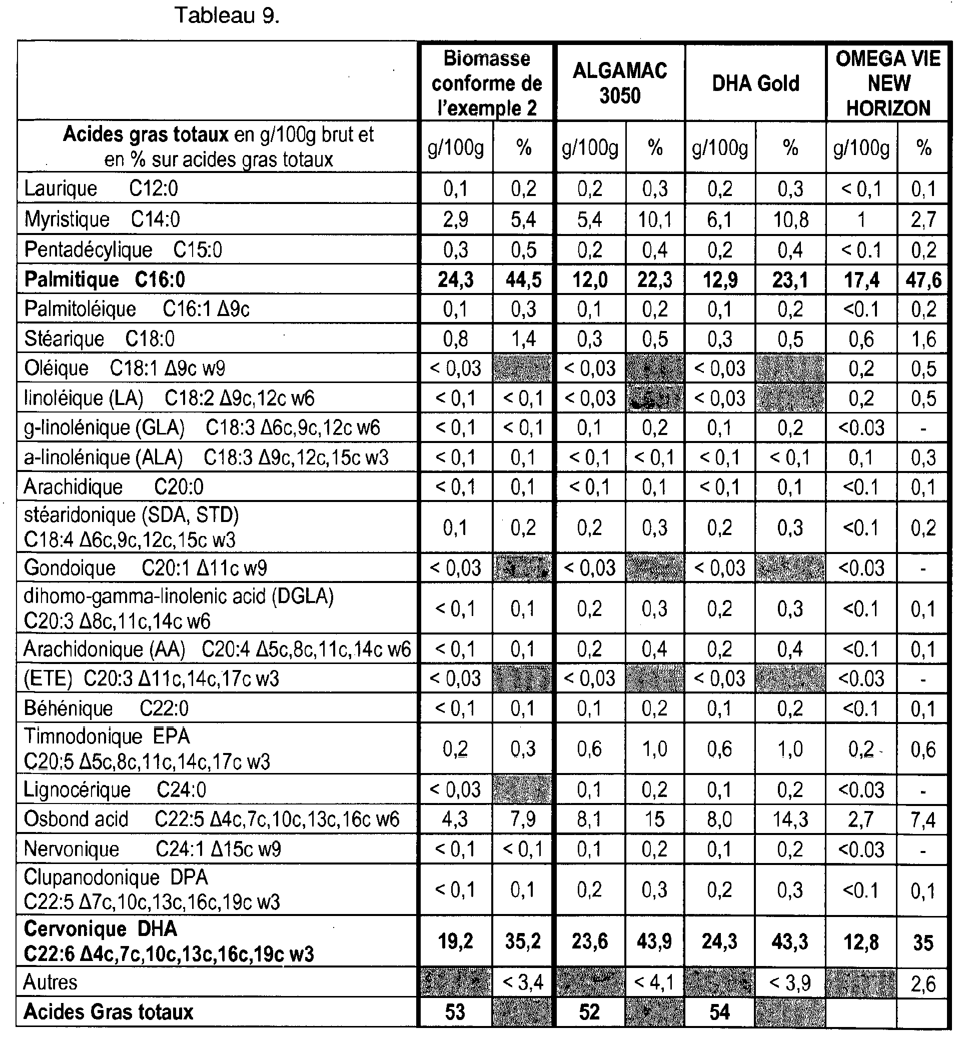

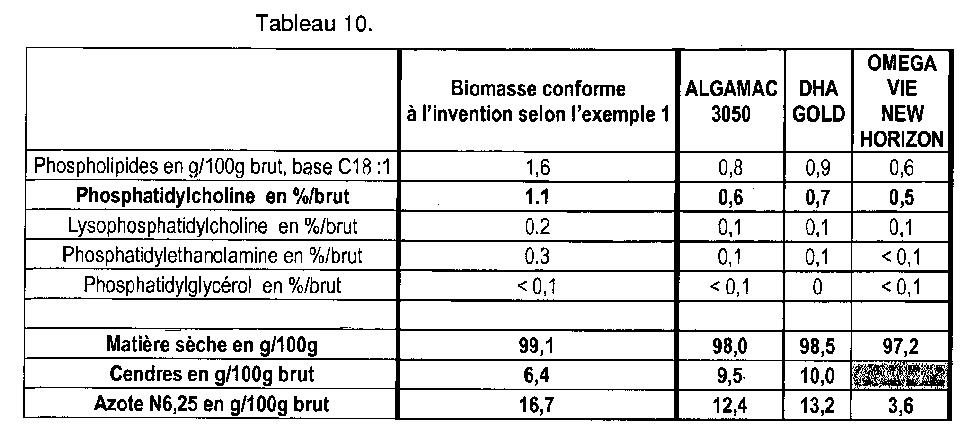

Il apparaît, à la lecture des résultats présentés ici que relativement au profil en acides gras :
- la biomasse selon l'invention présente une teneur en DHA légèrement inférieure à celle des biomasses riche en DHA du commerce, mais une teneur en acide palmitique double, ce qui constitue en quelque sorte l'empreinte de la biomasse de *Schizochytrium mangrovei* de l'invention ;
- par rapport à des huiles du commerce équivalentes en teneur en DHA et en acide palmitique, la biomasse selon l'invention présente une teneur en phospholipides double (plus particulièrement en phosphatidylcholine) ;
- une teneur en azote aminé bien supérieure.

## Revendications

1. Souche de *Schizochytrium mangrovei* déposée le 22 novembre 2012 auprès de la CNCM sous le numéro I-4702

2. Méthode de production d'une biomasse de microalgue contenant des composés lipidiques d'intérêt comprenant une culture de la souche selon la revendication 1 et la récupération de la biomasse riche en composés lipidiques d'intérêt.

3. Méthode selon la revendication 2, dans laquelle les composés lipidiques d'intérêt sont l'acide docosahexaénoïque (ou DHA) et l'acide palmitique.

4. Méthode selon la revendication 3, **caractérisée en ce que** la biomasse est préparée par la succession d'étapes suivantes :
o culture de la souche en conditions hétérotrophiques de manière à produire une biomasse présentant entre 35 et 40 % de DHA, exprimé en poids d'acides gras totaux et entre 40 et 50 % en poids d'acide palmitique, exprimé en poids d'acides gras totaux,
∘ récolte de la biomasse ainsi préparée
∘ séchage de ladite biomasse.

5. Utilisation de la biomasse produite par le procédé d'une quelconque des revendications 2 à 4 dans la préparation de compositions destinées aux domaines alimentaires.

6. Produit alimentaire comprenant la biomasse produite par le procédé d'une quelconque des revendications 2 à 4.

7. Méthode de préparation d'une souche de *Schizochytrium mangrovei* **caractérisée en ce que** la souche selon la revendication 1 est obtenue par mutagenèse ou par transformation génique et que l'on sélectionne une souche qui conserve la propriété de produire des teneurs en DHA et acide palmitique élevées.

8. Méthode selon la revendication 7, **caractérisée en ce que** la souche est sélectionnée si elle est capable de produire :
plus de 35 % de DHA, exprimés en poids d'acides gras totaux,
plus de 40 % d'acide palmitique, exprimés en poids d'acides gras totaux, et
entre 1 et 1,3 % de phosphatidylcholine, exprimé en poids de biomasse à 99 % de matière sèche.

## Patentansprüche

1. *Schizochytrium mangrovei* Stamm, hinterlegt am 22. November 2012 bei der CNCM unter der Nummer 1-4702.

2. Verfahren zur Produktion einer Lipidverbindungen von Interesse enthaltenden Mikroalgen-Biomasse, umfassend Kultivierung des Stamms gemäß Anspruch 1 und Wiedergewinnung der Biomasse, die reich an Lipidverbindungen von Interesse ist.

3. Verfahren gemäß Anspruch 2, wobei die Lipidverbindungen von Interesse Docosahexaensäure (oder DHA) und Palmitinsäure sind.

4. Verfahren gemäß Anspruch 3, **dadurch gekennzeichnet, dass** die Biomasse mithilfe der Abfolge nachfolgender Schritte hergestellt wird:
o Kultivierung des Stamms unter heterotrophen Bedingungen, um eine Biomasse zu produzieren, die zwischen 35 und 40% DHA, ausgedrückt als Gewichtsprozente der Gesamtfettsäuren, und zwischen 40 und 50 Gew.-% Palmitinsäure, ausgedrückt als Gewichtsprozente der Gesamtfettsäuren, aufweist,
∘ Ernte der auf diese Weise hergestellten Biomasse,
∘ Trocknung besagter Biomasse.

5. Verwendung der mit dem Verfahren nach einem der Ansprüche 2 bis 4 produzierten Biomasse in der Herstellung von Zusammensetzungen für den Nahrungsmittelbereich.

6. Nahrungsmittel, umfassend die mit dem Verfahren nach einem der Ansprüche 2 bis 4 produzierte Biomasse.

7. Verfahren zur Herstellung eines *Schizochytrium mangrovei* Stamms, **dadurch gekennzeichnet, dass** der Stamm gemäß Anspruch 1 mittels Mutagenese oder mittels Gentransformation erhalten wird und ein Stamm selektiert wird, der die Eigenschaft konserviert, erhöhte DHA- und Palmitinsäuregehalte zu produzieren.

8. Verfahren gemäß Anspruch 7, **dadurch gekennzeichnet, dass** der Stamm selektiert wird, der in der Lage ist:
mehr als 35% DHA, ausgedrückt als Gewichtsprozente der Gesamtfettsäuren,
mehr als 40% Palmitinsäure, ausgedrückt als Gewichtsprozente der Gesamtfettsäuren, und
zwischen 1 und 1,3% Phosphatidylcholin, ausgedrückt als Gewichtsprozente der Biomasse bei 99% Trockenmasse,
zu produzieren.

## Claims

1. A strain of *Shizochytrium mangrovei* deposited on November 22, 2012 at the CNCM under number I-4702.

2. A method for producing a biomass of microalga containing lipid compounds of interest comprising culture of the strain as claimed in claim 1 and recovery of the biomass rich in lipid compounds of interest.

3. The method according to claim 2, wherein the lipid compounds of interest are docosahexaenoic acid (or DHA) and palmitic acid.

4. The method according to claim 3, **characterized in that** the biomass is prepared by the sequence of the following steps:
o culturing the strain in heterotrophic conditions so as to produce a biomass having between 35 and 40% of DHA, expressed by weight of total fatty acids and between 40 and 50 wt% of palmitic acid, expressed by weight of total fatty acids,
∘ collecting the biomass thus prepared,
∘ drying said biomass.

5. Use of the biomass produced by the method according to any one of claims 2 to 4 in the preparation of compositions intended for the food sector.

6. A food product comprising the biomass produced by the method according to any one of claims 2 to 4.

7. A method of preparing a strain of *Schizochytrium mangrovei,* **characterized in that** the strain as claimed in claim 1 is obtained by mutagenesis or by gene transformation and that a strain is selected that conserves the property of producing high contents of DHA and palmitic acid.

8. The method according to claim 7, **characterized in that** the strain is selected if it is capable of producing:
more than 35% of DHA, expressed by weight of total fatty acids,
more than 40% of palmitic acid, expressed by weight of total fatty acids, and
between 1 and 1.3% of phosphatidylcholine, expressed by weight of biomass at 99% of dry matter.


### SEQUENCE LISTING

<110> ROQUETTE FRERES
<120> BIOMASSE DE LA MICROALGUE SCHIZOCHYTRIUM MANGROVEI ET SON PROCEDE DE PREPARATION
<130> B1551PC
<160> 1
<170> PatentIn version 3.3
<210> 1
   <211> 454
   <212> DNA
   <213> Schizochytrium mangrovei
<400> 1
   ggttttacat tgctctcatt ccgatagcaa aacgcataca cgcttcgcat cgatatttct     60
   cgtcctacct cgtggagtcc acagtgggta atttacgcgc ctgctgctat ccttggatat    120
   ggtagccgtc tctcaggctc cctctccgga gtcgagccct aactctccgt cacccgttat    180
   agtcaccgta gtccaataca ctaccgtcga caactgatgg ggcagaaact caaacgattc    240
   atcgaccaaa awagtcaatc tgctcaatta tcatgattca ccaataaaat cggcttcaat    300
   ctaataagtg cagccccata cagggctctt acagcatgta ttatttccag aattactgca    360
   ggtatccata taaaagaaac taccgaagaa attattactg atataatgag ccgttcgcag    420
   tctcacagta caatcgctta tacttacaca gcag                                454